# EUROPEAN PATENT APPLICATION

(11) **EP 1 380 578 A1**
(43) Date of publication of application: **14.01.2004**
(21) Application number: 02722705.7
(22) Date of filing: 18.04.2002
(51) Int. Cl.: C07D 311/24

(54) **PROCESS FOR PRODUCING NITRILE COMPOUND**

(30) Priority: 19.04.2001 JP 2001120945
(71) Applicant: ONO PHARMACEUTICAL CO., LTD., Osaka-shi, Osaka 541-8526 (JP)
(72) Inventor: QUAEDFLIEG, Peter, Jan, Leonard, Mario, NL-Geleen 6164 HB (NL); KUILMAN, Thijs, NL-Venlo 5925 AR (NL); JANSSEN, Johan, Willem, Joseph, Maria, NL-Velden 5941 JE (NL); HASHIMOTO, S, c/o Fukui Research Institute, Sakai-gun, Fukui 913-0032 (JP)
(74) Representative: Henkel, Feiler, Hänzel
(86) International application number: PCT/JP2002/003872
(87) International publication number: WO 2002/085880

(57) **Abstract**

Processes for the preparation of the nitrile compound of formula (1). 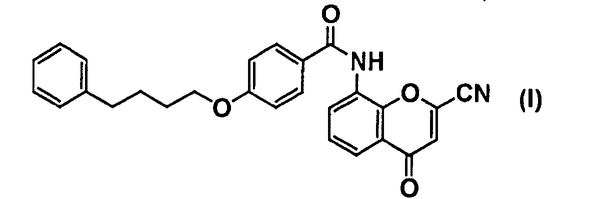 According to the present invention, the compound of formula (I) can be prepared cheaply and simply, therefore, the present invention is excellent as the industrial mass production method.

## Description

### TECHNICAL FIELD

The present invention relates to the process for the preparation of the nitrile compound which is useful as a pharmaceutical intermediate.

More particularly, the present invention relates to the process for the preparation of the nitrile compound of formula (I): 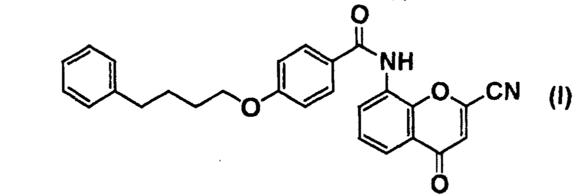 bv reacting the amide compound of formula (II): 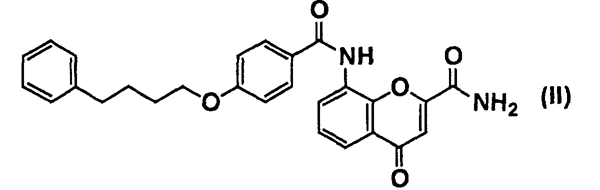 with cyanuric chloride.

### RELATED ARTS

The compound of formula (I) is important in the preparation of a pharmaceutical intermediate (See the specification of the Japanese Patent Kokai No. 61-50977 (European Patent No. 463638)).

As processes for the preparation of the compound of formula (I), for example, the specification of EP634409 discloses the process for the preparation of compounds of formula (A-3): 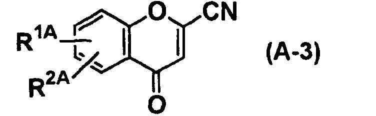 (wherein R^{1A} and R^{2A} are independently hydrogen, or R^{A}CONH wherein R^{A} is phenyl(C1-C20)alkoxyphenyl etc.)) by reacting the amide compound of formula (A-1): 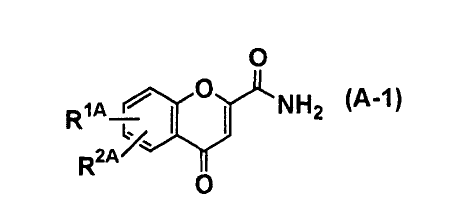 (wherein R^{1A} and R^{2A} are each as defined above), with a dehydrating agent (phosphorous pentoxide, phosphorous pentachloride, acetic anhydride, an ester of chloroformic acid, phosphorous oxychloride, phosgene etc.) in the presence of a pyridine compound of formula (A-2): 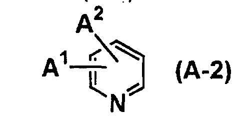 (wherein A¹ and A² are independently hydrogen or C1-C5 alkyl.). Only definitions of each symbols relevant to the present invention are described.

It is also disclosed that the compound of formula (A-3) can be additionally purified by dissolving the compound of formula (A-3) containing an impurities into an organic solvent insoluble or slightly soluble in water, pouring the solution obtained as above into water and simultaneously distilling the organic solvent with stirring, which precipitates to give the purified compound of formula (A-3) as a solid in the water phase.

Under such circumstances, as the result of thorough investigations in order to find an easier and cheaper method for industrial mass-production of the nitrile compound of formula (1) in higher purity, the present inventors have found out cyanuric chloride as cheaper dehydrating agent compared to phosphorous compounds that are generally used as dehydrating agents and that with cyanuric chloride no additional purification step is required as described above. Moreover, cyanuric chloride is very suitable for a method for industrial mass-production because it is more easily removed from the reaction product and it is more environmentally friendly, compared with phosphorous compounds, generally used as dehydrating agents.

Further, it has been found that the compound of formula (I) can be more easily isolated in a pure form by crystallization or precipitation using alcoholic solvents. Under such conditions, no cyanuric chloride or its alkoxy-substituted congeners are precipitated together with the desired compound of formula (I). Thus, the obtained compound of formula (I) contains only few impurities and is obtained as off-white to white crystals, and then the present invention has been accomplished.

### DISCLOSURE OF THE INVENTION

The present invention relates to a process for the preparation of the nitrile compound of formula (I): 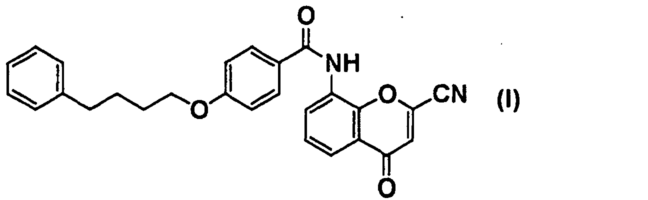 which is characterized by reacting the amide compound of formula (II): 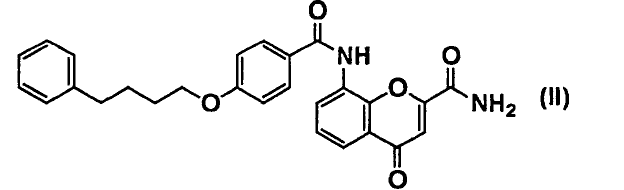 with cyanuric chloride.

The preparation of the nitrile compound of formula (I) from the amide compound of formula (II), for example, can be carried out by reacting with cyanuric chloride as a dehydrating agent in a polar aprotic organic solvent (e.g. N,N-dimethyl-formamide etc.) at about -30 to 50°C, and then if necessary, subjecting to work up by adding an alcoholic solvent (e.g. methanol, ethanol etc.).

The preferred polar aprotic organic solvent is N,N-dimethylformamide.

The reaction temperature is preferably about -20 to 20°C.

Preferred alcoholic solvents are methanol. and ethanol.

### BEST MODE FOR CARRYING OUT THE INVENTION

The following examples illustrate the present invention, but not limit the present invention.

### Example 1

### The preparation of N-(2-cyano-4-oxochromen-8-yl)-4-(4-phenylbutoxy)phenylcarboxamide

4-Oxo-8-((4-(4-phenylbutoxy)phenyl)carbonylamino)chromene-2-carboxamide (7.67 g) was dissolved in N,N-dimethylformamide (70 mL), and cyanuric chloride (7.55 g) was added thereto at -20°C, and the reaction mixture was stirred at 5 to 10°C for 4 hours. To the reaction mixture, methanol (100 mL) was added dropwise at 10 to 15°C. The precipitated solid material was filtered off at 15°C, and washed with cold water (50 mL x 2) and cold methanol (50 mL x 2) successively, and dried at ambient temperature to give a white powder of N-(2-cyano-4-oxochromen-8-yl)-4-(4-phenylbutoxy)phenylcarboxamide (yield 75%).

### Example 2

### The preparation of N-(2-cyano-4-oxochromen-8-yl)-4-(4-phenylbutoxy)phenylcarboxamide

4-Oxo-8-((4-(4-phenylbutoxy)phenyl)carbonylamino)chromen-2-yl-carboxamide (7.67 g) was dissolved in N,N-dimethylformamide (70 mL), and cyanuric chloride (7.55 g) was added thereto at -20°C, and the reaction mixture was stirred at 5 to 10°C for 8 hours. To the reaction mixture, methanol (200 mL) was added dropwise at 15°C. The precipitated solid material was filtered off at 10°C, and washed with cold water (50 mL x 2) and cold methanol (50 mL x 2) successively, and dried at ambient temperature to give a white powder of N-(2-cyano-4-oxochromen-8-yl)-4-(4-phenylbutoxy)phenylcarboxamide (yield 85%).

### INDUSTRIAL APPLICABILITY

According to the present invention, the nitrile compound of formula (I) can be prepared in a highly pure form from the amide compound of formula (II), cheaply and simply. Therefore, the present method is excellent as a method of the industrial mass-production method.

## Claims

1. A process for the preparation of the nitrile compound of formula (I): 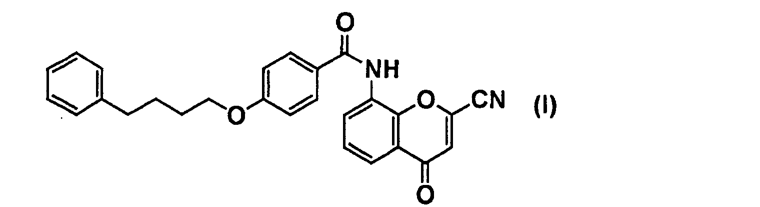 which comprises reacting the amide compound of formula (11): 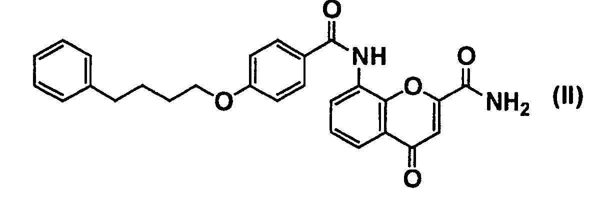 with cyanuric chloride.

2. The process according to claim 1, which is **characterized by** work up by adding alcohol solvent(s).
